Europäisches Patentamt

(19) European Patent Office

Office européen des brevets

(11) Veröffentlichungsnummer: **0 127 723**
**B1**

(12) **EUROPÄISCHE PATENTSCHRIFT**

(45) Veröffentlichungstag der Patentschrift:
07.10.87

(21) Anmeldenummer: 84100626.5

(22) Anmeldetag: 23.01.84

(51) Int. Cl.⁴: **B 01 J 23/40,** B 01 J 23/56,
B 01 J 23/74, B 01 J 23/76,
C 07 C 5/05

(54) **Katalysator zur selektiven Hydrierung von mehrfach ungesättigten organischen Verbindungen.**

(30) Priorität: 06.06.83 DE 3320388

(43) Veröffentlichungstag der Anmeldung:
12.12.84 Patentblatt 84/50

(45) Bekanntmachung des Hinweises auf die Patenterteilung:
07.10.87 Patentblatt 87/41

(84) Benannte Vertragsstaaten:
DE FR GB IT

(56) Entgegenhaltungen:
DE - A - 2 025 411
DE - A - 2 713 457
GB - A - 948 861
US - A - 2 360 555
US - A - 2 887 517
US - A - 4 264 421

Experimental methods in catalytic research,
R.B.Anderson Academic Press, New York/London
(1968), p.81
CHEMICAL ABSTRACTS, Band 80, Nr. 19, 13. Mai 1974,
Seite 378, Zusammenfassung Nr. 1080711, COLUMBUS,
OHIO (US)
CHEMICAL ABSTRACTS, Band 81, Nr. 1, 8. Juli 1974,
Seite 278, Zusammenfassung Nr 3461j, COLUMBUS,
OHIO (US)

(73) Patentinhaber: SÜD-CHEMIE AG, Lenbachplatz 6,
D-8000 München 2 (DE)

(72) Erfinder: Stadler, Karl-Heinz, Dr., Dipl.-Chem., Steinerne
Furt 6, D-8900 Augsburg (DE)
Erfinder: Kochloefl, Karel, Dr., Dipl.-Ing., Kreuzstrasse 2,
D-8052 Moosburg (DE)

(74) Vertreter: Reitzner, Bruno, Dr., Patentanwälte Dipl.-Ing.
R. Splanemann Dr. B. Reitzner Tal 13,
D-8000 München 2 (DE)

## Beschreibung

Die Erfindung betrifft ein Verfahren zur selektiven katalytischen Hydrierung von mehrfach ungesättigten organischen Verbindungen, insbesondere von mehrfach ungesättigten Kohlenwasserstoffen.

Eine Reihe von selektiven Hydrierungsreaktionen ist technisch von grossem Interesse. Besonders in der Petrochemie nimmt die Olefinchemie einen breiten Raum ein. Technisch wichtig ist etwa die Diolefinentfernung aus Pyrolysebenzin. Vor allem zu erwähnen ist die selektive Hydrierung von Butadienen. Erwähnenswert ist ferner die selektive Hydrierung von Isopren oder Cyclopentadien.

Besonders schwierig gestaltet sich die selektive Hydrierung von Dienen mit isolierten Doppelbindungen, besonders von höheren Homologen dieser Diene. Sie verhalten sich bei der Hydrierung üblicherweise wie Monoolefine, d.h. sie werden sofort zum entsprechenden Alkan perhydriert. Will man eine selektive Hydrierung erreichen, die auf der Stufe des Monoolefins stehen bleibt, so sollte der Reaktion nach der Lehrmeinung eine Doppelbindungsisomerisierung zu einem konjugierten Dien vorausgehen. Diese Isomerisierung wird in der Regel durch die Oberflächeneigenschaft des Trägermaterials, etwa seine Acidität, beeinflusst.

Die am weitesten verbreiteten und wirtschaftlichsten Katalysatorsysteme für die selektive Hydrierung enthalten als katalytisch wirksame Substanzen d-Metalle der Gruppe VIII des Periodensystems auf einem inerten oxidischen Trägermaterial, insbesondere auf Aluminiumoxid. Durch eine partielle Vergiftung der Metallkomponente, z.B. der Palladiumkomponente, mit Pb, Zn, Hg, Cd, Sn usw. kann die Selektivität bezüglich der Bildung von Alkenen aus Diolefinen erhöht werden (sogenannte «Lindlar-Katalysatoren», vgl. H. Lindlar, Helv.Chim. Acta, 35, 446 (1952) oder allgem. G.C. Bond, «Catalysis by Metals», Academic Press, London, New York (1962), 99 und 297). Andererseits ist die richtige Dosierung bei der Zugabe des Katalysatorgiftes problematisch. Oft werden Katalysatoren erhalten, die entweder unselektiv oder völlig inaktiv sind.

Es existiert ferner eine Anzahl kommerzieller Katalysatoren zur selektiven Hydrierung; diese enthalten Palladium in Konzentrationen von 0,03 bis 0,5 Gew.-% auf unterschiedlichen Aluminiumoxiden. Auch die spezifische Vergiftung mit Cr wird angewendet, wobei etwa 0,5 Gew.-% Cr zugesetzt werden. Aber auch diese Katalysatoren sind oft zu wenig selektiv oder bei guter Selektivität zu wenig aktiv.

Schliesslich beschreibt die US-A-4 264 421 pulverförmige Katalysatoren auf der Basis von n-halbleitenden Oxiden, wie Titandioxid, Wolframtrioxid und Eisenoxid bzw. auf der Basis von anderen n-halbleitenden Substanzen, wie Galliumarsenid und Strontiumtitanat, auf die Platin aufgebracht ist. Diese Katalysatoren werden jedoch nur zur Decarboxylierung von gesättigten Carbonsäuren sowie für die thermische Hydrierung von Benzol und Cyclohexen zu Cyclohexan verwendet. Es finden sich keine Angaben über das Wasserstoff-Chemisorptionsvermögen bzw. darüber, dass durch die Auswahl eines bestimmten Wasserstoff-Chemisorptionsvermögens eine Selektivität zur Hydrierung von mehrfach ungesättigten organischen Verbindungen erreicht werden kann.

Es wurde nun überraschenderweise ein Verfahren zur selektiven katalytischen Hydrierung von mehrfach ungesättigten organischen Verbindungen, insbesondere von Olefin-Diolefin-Mischungen mit einem Anteil an Diolefinen mit isolierten Doppelbindungen gefunden, das gekennzeichnet ist durch die Verwendung eines Katalysators, enthaltend

(A) eine Metallkomponente aus einem oder mehreren Elementen der Gruppe VIII des Periodensystems auf

(B) einem Trägermaterial auf der Basis

(b$_1$) eines oder mehrerer n-halbleitender Oxide eines oder mehrerer Elemente aus den Nebengruppen IVb, Vb und VIb des Periodensystems oder des Thoriums oder Cers, bzw. auf der Basis

(b$_2$) eines oder mehrerer n-halbleitender Mischoxide der Formel Me$_2$Me$_1$ (O)$_x$, worin Me$_1$ ein Element aus der in (b$_1$) definierten Gruppe, Me$_2$ ein Erdalkalimetall oder ein von Me$_1$ verschiedenes Element aus der in (b$_1$) definierten Gruppe und x die Anzahl der zur Absättigung von Me$_1$ und Me$_2$ bis zum Bereich eines n-halbleitenden Zustandes erforderlichen Sauerstoffatome bedeuten;

wobei das Wasserstoff-Chemisorptionsvermögen, ausgedrückt als das Atomverhältnis zwischen chemisorbierten Wasserstoffatomen und an der Oberfläche der Metallteilchen befindlichen Metallatomen der Metallkomponente (A) (H/Me$_A$) mindestens 0,6:1 beträgt.

Die Nebengruppen IVb, Vb und IVb des Periodensystems sind nach «Handbook of Chemistry and Physics» 58. Auflage, 1978/79 definiert.

Das Wasserstoff-Chemisorptionsvermögen (H/Me$_A$) kann nach dem in «Experimental Methods in Catalytic Research», R.B. Anderson, Academic Press, New York/London, 1968, Seite 81 beschriebenen volumetrischen Verfahren bestimmt werden. Hierbei lässt man ein bestimmtes Volumen Wasserstoff bei Raumtemperatur und Atmosphärendruck auf den Katalysator einwirken und stellt die Volumenabnahme fest. Dann wird der physikalisch sorbierte Anteil des Wasserstoffs bis zu einem Druck von 10$^{-5}$ mbar abgepumpt. Der verbleibende Anteil, bezogen auf die der Wasserstoffadsorption zugänglichen Oberflächenatome der Metallkomponente (A) (Atomverhältnis), entspricht dem Wasserstoff-Chemisorptionsvermögen des Katalysators.

Der erfindungsgemässe Katalysator ist insbesondere durch ein Wasserstoff-Chemisorptionsvermögen (H/Me$_a$) von 0,75 bis 1:1 gekennzeichnet.

Wesentlich für die Erfindung ist die Tatsache, dass das erfindungsgemäss verwendete n-halbleitende Trägermaterial nicht als chemisch inert aufzufassen ist. Es bildet sich nämlich bereits während der Herstellung oder während des Einsatzes eine Metall-Träger-Wechselwirkung aus.

Wesentlich für die Erfindung ist die Tatsache, dass das erfindungsgemässe n-halbleitende Trägermaterial nicht als chemisch inert aufzufassen ist. Es bildet sich nämlich bereits während der Herstellung oder während des Einsatzes eine Metall-Träger-Wechselwirkung aus.

Es ist bekannt, dass Metall-Träger-Wechselwirkungen die Adsorptionseigenschaften eines solchen Systems wesentlich beeinflussen. So beschreibt z.B. die DE-A-27 13 457 Katalysatormischungen mit verringerter Wasserstoff-Chemisorptionskapazität aus einer katalytischen Metallkomponente, bestehend aus einem Metall der Gruppe VIII des Periodensystems auf einem Übergangsmetalloxid aus der Gruppe Nioboxid, Tantaloxid, Vanadinoxid, Titanoxid, Zirkonoxid, Hafniumoxid oder Mischungen derselben. Als Trägermaterialien werden aber auch Erdalkalititanate beschrieben.

Diese Katalysatoren werden als Katalysatoren mit «starken Wechselwirkungen zwischen Metall und Träger» (strong metal support interaction catalysts, abgekürzt SMSI-Katalysatoren) bezeichnet. Der SMSI-Effekt wird durch eine intensive Reduktionsbehandlung des Katalysator-Vorläufers mit Wasserstoff bei Temperaturen zwischen 475 und 775 K erreicht. Die so erhaltenen Katalysatoren zeichnen sich durch ein stark vermindertes Wasserstoff-Chemisorptionsvermögen $(H/Me_A)$ aus, das maximal etwa 2% des Ausgangswertes beträgt. Bedingt durch dieses stark verminderte Chemisorptionsvermögen für Wasserstoff zeigen diese Katalysatoren ein verändertes Aktivitäts- und Selektivitätsverhalten bezüglich der Dehydrocyclisierung von n-Heptan oder der Ethan-Hydrogenolyse. In weiteren Arbeiten wurde festgestellt, dass $Ni/TiO_2$-Katalysatoren die analog dem in der DE-A-27 13 457 beschriebenen Verfahren hergestellt wurden, eine hohe Aktivität und Selektivität bei der Methanisierung von CO aufweisen (M.A. Vannice und R.L. Garten, J. Ctal. 56, 236 (1979) ). Diese Katalysatoren sind jedoch für eine selektive Hydrierung von mehrfach ungesättigten organischen Verbindungen nicht geeignet.

Überraschenderweise wurde nun gefunden, dass die erfindungsgemäss verwendeten Katalysatoren, die sich von den genannten Katalysatoren im wesentlichen dadurch unterscheiden, das ihr Wasserstoff-Chemisorptionsvermögen $(H/Me_A)$ mindestens 0,6:1 beträgt, zur selektiven Hydrierung von mehrfach ungesättigten organischen Verbindungen sehr gut eignen. Bei diesen Katalysatoren treten aufgrund der Tatsache, dass das Trägermaterial ein n-Halbleiter ist, Metall-Trägermaterial-Wechselwirkungen auf, die aber nicht als SMSI-Effekte im Sinne der DE-A-27 13 457 zu verstehen sind. Wahrscheinlich handelt es sich bei den erfindungsgemäss verwendeten Katalysatoren um Metall-Träger-Systeme, bei denen über eine Angleichung des Fermi-Niveaus eine «charge-transfer»-Wechselwirkung auftritt (vgl. G.-M. Schwab, Elektronik der Trägerkatalysatoren, Catalysis 25, 106 (1972) ), die sowohl die Adsorptionsreaktion quantitativ wie qualitativ beeinflusst, als auch die chemischen und physikalischen Eigenschaften des beim katalytischen Prozess auftretenden Adsorbats bestimmt. Eine solche Wechselwirkung sollte im Fall von $TiO_2$, besonders bei gleichzeitiger Wasserstoffadsorption, zur Bildung $Ti^{3+}$-ähnlicher «Oberflächenzentren» (surface sites) führen, die ihrerseits einen selektivitätsfördernden Einfluss ausüben können, etwa dadurch, dass sie als dienophile Koordinationszentren wirken.

Der erfindungsgemäss verwendete Katalysator ist insbesondere dadurch gekennzeichnet, dass seine Metallkomponente (A) ein Edelmetall aus der Gruppe VIII des Periodensystems, insbesondere Palladium, und sein n-halbleitendes Trägermaterial

(B) entweder

($b_1$) ein Oxid eines der Elemente aus den Nebengruppen IVb, Vb und VIb des Periodensystems, insbesondere von Ti, Zr, V, Nb, Ta, Cr, Mo oder W, oder des Thoriums; oder

($b_2$) $SrTiO_3$, $BaTiO_4$ oder $TiNb_2O_7$ darstellt.

Hierbei wird insbesondere das System $Pd/TiO_2$ bevorzugt.

Ferner kann der Katalysator neben dem n-halbleitenden Trägermaterial ein inertes, nicht n-halbleitendes Füllmaterial, bevorzugt Aluminiumoxid oder Siliciumoxid, Magnesiumoxid, Calciumcarbonat oder dergl. enthalten. Mit Hilfe des inerten Füllmaterials wird die Formbarkeit des Katalysators verbessert. Diese ist wichtig, weil ein pulverförmiger Katalysator im Reaktor einen erhöhten Strömungswiderstand hat und zu einer erhöhten Staubbildung Anlass gibt. Auch das Diffusionsverhalten der am Katalysator reagierenden Substanzen wird durch die Eigenschaften des inerten Füllmaterials verbessert. Schliesslich bewirkt das inerte Füllmaterial einen Verdünnungseffekt, wodurch etwa auftretende exotherme Temperaturspitzen abgebaut werden.

Es muss lediglich durch geeignete Verfahrensführung sichergestellt werden, dass die Metallkomponente nur an den n-halbleitenden Trägermaterialien, nicht aber an den inerten Füllmaterialien adsorbiert ist, damit die schwache Wechselwirkung zwischen der Metallkomponente und dem n-halbleitenden Trägermaterial auftritt.

Die erfindungsgemäss verwendeten Katalysatoren können zur Erzielung des als wesentlich erachteten, nur leicht verminderten Wasserstoff-Chemisorptionsvermögens grundsätzlich nach zwei Verfahrensvarianten hergestellt werden.

Nach der ersten Verfahrensvariante geht man im allgemeinen so vor, dass man eine reduzierbare Verbindung der Metallkomponente (A), z.B. eine Lösung eines Edelmetallsalzes, auf eine Vorstufe des n-halbleitenden Trägermaterials (B) aufbringt und unter Bedingungen, bei denen das Wasserstoff-Chemisorptionsvermögen $(H/Me_A)$ des erhaltenen Katalysators auf mindestens 0,6:1 gehalten wird, zur Metallkomponente (A) reduziert.

Diese Reduktion wird vorzugsweise in einer reduzierenden Atmosphäre bei Temperaturen durchgeführt, bei denen das Wasserstoff-Chemisorptionsvermögen (H/Me$_A$) nicht unter 0,6:1 absinkt. Diese Temperaturen sind von dem verwendeten System Metallkomponente/Trägerstoff, dem Reduktionsmittel und der Zeit abhängig. Beispielsweise wird die Reduktion mit Wasserstoff systemabhängig unterhalb der folgenden Maximaltemperaturen über 3 Stunden durchgeführt:

| System | Temp (K) |
|---|---|
| Pd/TiO$_2$ | 470 |
| Pd/SrTiO$_3$ | 800 |
| Pd/BaTiO$_3$ | 800 |
| Pd/ZrTiO$_4$ | 500 |
| Pd/TiNb$_2$O$_7$ | 470 |
| Pd/CeO$_2$ | 600 |

Verglichen mit den in der DE-A-27 13 457 angegebenen Bedingungen wird also die Reduktion bei verhältnismässig milden Bedingungen durchgeführt.

Die Reduktion der reduzierbaren Verbindung der Metallkomponente (A) kann aber auch mit einem Reduktionsmittel, wie Formaldehyd oder Natriumformiat, in flüssiger Phase, vorzugsweise in wässrigem Medium, durchgeführt werden. Dies stellt eine besonders milde Form der Reduktionsbehandlung dar.

Die Reduktion der reduzierbaren Verbindung der Metallkomponente (A) kann aber auch in an sich bekannter Weise photochemisch bei Umgebungstemperatur durchgeführt werden, was eine weitere milde Art der Reduktionsbehandlung darstellt (vgl. Dissertation Stadler, Universität München, 1982).

Nach der anderen allgemeinen Verfahrensvariante kann der erfindungsgemäss verwendete Katalysator dadurch hergestellt werden, dass man eine reduzierbare Verbindung der Metallkomponente (A) auf eine Vorstufe des n-halbleitenden Trägermaterials (B) aufbringt und unter Bedingungen, bei denen das Wasserstoff-Chemisorptionsvermögen des erhaltenen Katalysators (H/Me$_a$) unterhalb 0,6:1 absinkt, zur Metallkomponente (A) reduziert und den erhaltenen Katalysator oxidiert, bis das Wasserstoff-Chemisorptionsvermögen (H/Me$_A$) den Wert von 0,6:1 wieder erreicht oder überschritten hat. Diese Variante hat den Vorteil, dass bei der Reduktion keine besonderen Vorsichtsmassnahmen (Einhaltung bestimmter Reduktionstemperaturen und -zeiten) getroffen werden müssen.

Durch die Oxidationsbehandlung wird der Katalysator, der durch die Reduktion unter scharfen Bedingungen in den für den vorliegenden Zweck unerwünschten SMSI-Zustand übergeführt wurde, in den für eine selektive Hydrierung von organischen Verbindungen erwünschten Zustand übergeführt, bei dem nur eine schwache Wechselwirkung zwischen der Metallkomponente und dem Trägermaterial existiert.

Soll aus den vorstehend angegebenen Gründen ein nicht n-halbleitendes Füllmaterial zugesetzt werden, so setzt man dieses dem bereits reduzierten Katalysator zu, um sicherzustellen, dass die reduzierte Metallkomponente nicht etwa am inerten Füllmaterial adsorbiert wird. Es wird also beispielsweise der reduzierte Katalysator mit Aluminiumoxid vermischt. Bei einer etwaigen weiteren thermischen Behandlung sollte die Bildung von Mischoxiden mit Aluminiumoxid oder die Bildung von mit Al$^{3+}$ dotierten Oxiden ausgeschlossen werden, da auf diese Weise der n-Halbleiter-Charakter der verwendeten Trägermaterialien geschwächt würde.

Setzt man ein inertes Füllmaterial zu, so führt man die vorherige Reduktionsbehandlung vorzugsweise nach der zweiten Verfahrensvariante durch, d.h. man reduziert unter schärferen Bedingungen, damit die Metallkomponente stärker an das n-halbleitende Trägermaterial gebunden wird und nicht auf das inerte Trägermaterial wandert. Bei der anschliessenden Oxidationsbehandlung zur Wiederherstellung des Wasserstoff-Sorptionsvermögens ist eine Wanderung der Metallkomponente nicht mehr zu befürchten.

Als Metallkomponente (A) wird vorzugsweise ein Edelmetall verwendet, dessen Gehalt zwischen etwa 0,01 und 5%, bezogen auf das Gewicht des fertigen Katalysators, beträgt.

Verwendet man als inertes Füllmaterial Aluminiumoxid, so kann ein Teil davon zur besseren Formbarkeit (Tabletten, Strangpresskörper usw.) in Form von Aluminiumstearat zugesetzt werden. Auch die Titandioxidkomponente kann zum Teil in Form von Titanstearat zugesetzt werden. Diese Verbindungen liefern bei der Oxidationsbehandlung die entsprechenden Oxide.

Die Oxidationsbehandlung kann bei Temperaturen bis zu etwa 775 K erfolgen, insbesondere bei Katalysatoren mit Titandioxid als Trägermaterial.

Der Oxidationsbehandlung kann sich eine weitere Reduktionsbehandlung anschliessen, die jedoch so zu führen ist, dass eine Umwandlung des Katalysators in den SMSI-Zustand vermieden wird.

Eine weitere Möglichkeit der Herstellung der erfindungsgemäss verwendete Katalysatoren, am Beispiel eines TiO$_2$-Katalysators erläutert, besteht im Auffällen eines Titanoxidhydrats auf ein inertes, vorgeformtes, pulveriges oder granuliertes Trägermaterial. Dies kann beispielsweise durch Hydrolyse von TiCl$_4$, TiOSO$_4$, Ti-Isopropylat, TiCl$_3$ oder anderen hydrolysierbaren Titanverbindungen geschehen. Auch die Behandlung eines vorgeformten Trägermaterials, etwa von sphärischem Al$_2$O$_3$ genügend hoher Makroporosität, mit einem Titandioxidsol, hergestellt durch Hydrolyse etwa von Ti-Isopropylat, führt zur Vorstufe der erfindungsgemässen Katalysatoren. Geeignet sind auch titanoxidhaltige Träger, die beispielsweise nach der DE-C-26 58 569 oder nach der DE-A-24 46 496 hergestellt werden. Ein nachfolgender Calcinierschritt unter einer oxidierenden Atmosphäre führt zu den gewünschten Trägermaterialien. Die Aufbringung der Metallkomponente, vorzugsweise der Edelmetallkomponente, erfolgt in der schon beschriebenen Weise.

Schliesslich kann es sich zur Verbesserung der Selektivität als vorteilhaft erweisen, den Katalysator mit phosphat- oder fluoridhaltigen Lösungen zu imprägnieren. Man verwendet zu diesem Zweck beispielsweise eine $H_3PO_4$-Lösung oder eine NaF-Lösung. Vorzugsweise wird diese Behandlung mit $TiO_2$-Katalysatoren durchgeführt. Die spezifische Phosphatadsorption am $TiO_2$ bedingt eine Zunahme der Oberflächenacidität.

Das erfindungsgemässe Verfahren kann insbesondere zur selektiven Hydrierung von mehrfach ungesättigten Kohlenwasserstoffen angewendet werden.Besonders gut lassen sich Diolefine mit isolierten Doppelbindungen bzw. Diene mit kumulierten und/oder konjugierten Doppelbindungen selektiv hydrieren, wobei die Diolefine bzw. Diene auch in beliebigen Kohlenwasserstoffgemischen enthalten sein können, und zwar auch in alkenhaltigen Gemischen. Hervorzuheben ist, dass die Hydrierung der Diolefine zu den entsprechenden Alkenen auch dann mit hohen Ausbeuten durchführbar ist, wenn das Einsatzmaterial nicht-konjugierte Diolefine mit isolierten Doppelbindungen mit Kettenlängen von 10 bis 15 oder mehr Kohlenstoffatomen enthält.

Ferner lässt sich das erfindungsgemässe Verfahren auch zur selektiven Hydrierung von ungesättigten organischen Verbindungen anwenden, die neben der C=C-Doppelbindung auch C=O- und C=N-Gruppen enthalten. Beispiele für diese Verbindungen sind ungesättigte Ester, Aldehyde, Säureanhydride und Nitrile, wie Acrolein, Acryl- und Methacrylsäureester und -nitrile sowie Maleinsäureanhydrid.

Ein weiterer Vorteil besteht darin, dass die erfindungsgemäss verwendeten Katalysatoren mit einem vergleichbaren bzw. bis zu 50% geringeren Edelmetallgehalt höhere oder wenigstens gleiche Aktivitäten und vor allem höhere Selektivitäten als vergleichbare Katalysatoren mit inerten Trägermaterialien erreichen.

Die nachstehenden Beispiele 1 bis 10 erläutern die Herstellung einiger erfindungsgemäss verwendeter Katalysatoren. In den Vergleichsbeispielen 1 bis 3 ist die Herstellung einiger bekannter Katalysatoren angegeben. Einige physikalische und chemische Daten der erfindungsgemäss verwendeten Katalysatoren und der Vergleichskatalysatoren, einschliesslich einiger handelsüblicher Katalysatoren, sind in Tabelle I angegeben. Die Tabellen II und III enthalten Daten, die es gestatten, die Überlegenheit der erfindungsgemäss verwendeten Katalysatoren gegenüber den Vergleichskatalysatoren zu beurteilen. Zu diesem Zweck wurde die selektive Hydrierung der Dienkomponente in Mischungen aus 1-Hexan mit 1,5-Hexadien herangezogen, ferner die selektive Hydrierung der Dien- und Diolefinkomponente (etwa 10%) in entsprechenden $C_{10}$-Mischungen.

Beispiel 1

Titandioxid (spez. Oberfläche = 50 $m^2$/g, 70% Anatas, 30% Rutil) wird mit einer wässrigen $PdCl_2$-Lösung bis zu einem Pd-Gehalt von 0,5 Gew.-% imprägniert und im $H_2$-Strom 3 Stunden bei 675–723 K reduziert. Dieser Reduktionsschritt unterbleibt oder wird bei niedrigeren Temperaturen (unterhalb 470 K) durchgeführt, wenn anschliessend nicht in oxidierender Atmosphäre calciniert wird. Der Katalysator kann bereits in der erhaltenen Pulverform eingesetzt werden.

Zur Verdünnung wird der Katalysator mit AlO (OH) als Füllmaterial (spez. Oberfläche = 300 $m^2$/g, Porenvolumen = 0,46 ml/g) im Gewichtsverhältnis 1:1 gründlich gemischt, worauf 3 bis 4 Gew.-% Al-Stearat oder Ti-Stearat zugesetzt werden, um die Formgebung zu erleichtern. Nach dem Tablettieren des Materials (4,5×4,5 mm) wird etwa 3 Stunden bis zu 775 K unter Luftzutritt calciniert. Der Pd-Gehalt beträgt nun 0,25 Gew.-%. Für die Durchführung der Versuche, deren Ergebnisse in Tabelle II angegeben sind, wurden die Tabletten nochmals zerrieben, d.h. der Katalysator wurde als Pulver eingesetzt.

Beispiel 2

Titandioxid (spez. Oberfläche = 50 $m^2$/g, 70 Gew.-% Anatas, 30 Gew.-% Rutil) wird mit $Sr(NO_3)_2$ oder $Sr(OH)_2$ imprägniert, so dass ein Produkt mit einem Gehalt von 10 Gew.-% SrO erhalten wird. Dieses Produkt wird mit wässriger $PdCl_2$-Lösung imprägniert und bei 675–725 K im Wasserstoffstrom reduziert. Anschliessend wird analog Beispiel 1 weitergearbeitet. Der Katalysator enthält entsprechend der eingesetzten Sr-Menge $SrTiO_3$. Sein Pd-Gehalt beträgt 0,25 Gew.-%.

Beispiel 3

Die Arbeitsweise von Beispiel 1 wird mit der Abweichung wiederholt, dass ein Titandioxidhydrat einer spezifischen Oberfläche von 200 $m^2$/g eingesetzt wird.

Beispiel 4

Die Arbeitsweise von Beispiel 1 wird mit der Abweichung wiederholt, dass als Trägermaterial ein Cerdioxid mit einer spezifischen Oberfläche von 60 $m^2$/g verwendet und die Reduktion bei 600 K durchgeführt wird.

Beispiel 5

Die Arbeitsweise von Beispiel 1 wird mit der Abweichung wiederholt, dass als Trägermaterial ein Niobpentoxid mit einer spezifischen Oberfläche von 117 $m^2$/g verwendet und die Reduktion bei 670 K durchgeführt wird. Anschliessend wird unter Luftzutritt calciniert.

Beispiel 6

$TiO_2$ (50 $m^2$/g) wird mit einer $PdCl_2$-Lösung (0,25 Gew.-%) imprägniert. Das erhaltene Produkt wird 3 Stunden bei 700 K mit $H_2$ reduziert und anschliessend mit 0,3 m $H_3PO_4$-Lösung im-

prägniert. Der Phosphatgehalt beträgt 0,5 bis 2 Gew.-%. Das imprägnierte Produkt wird wie im Beispiel 1 angegeben, unter Luftzutritt calciniert.

Beispiel 7

Die Arbeitsweise von Beispiel 6 wird mit der Abweichung wiederholt, dass der Katalysator mit 0,3 m NaF-Lösung imprägniert wird. Die Natriumionen werden anschliessend mit Wasser weitgehend ausgewaschen. Der Fluoridgehalt beträgt 0,1 bis 2 Gew.-%.

Beispiel 8

Die Arbeitsweise von Beispiel 1 wird mit der Abweichung wiederholt, dass dem Produkt vor dem Tablettieren neben Aluminiumoxid noch 1 Gew.-% WO$_3$ zugemischt werden.

Beispiele 9 und 10

2 g TiO$_2$ mit einer spezifischen Oberfläche von 50 m$^2$/g werden in 500 ml eines Alkohols, z.B. Ethanol, suspendiert; dann wird soviel wässrige PdCl$_2$-Lösung zugegeben, dass der Pd-Gehalt, bezogen auf 1 g TiO$_2$, 0,56 bzw. 1,19 Gew.-% beträgt. Die Suspension wird zur Reduktion der Metallkomponente unter Inertgas mit UV-Licht bestrahlt. Die so erhaltenen Katalysatoren werden abfiltriert und getrocknet.

Neben den zu Vergleichszwecken herangezogenen handelsüblichen Katalysatoren wurden, ebenfalls zu Vergleichszwecken, noch einige Pdhaltige Katalysatoren auf nicht n-halbleitenden Trägermaterialien verwendet, deren Herstellung in den nachstehenden Vergleichsbeispielen 1 bis 3 angegeben ist.

Vergleichsbeispiel 1

Hydratisiertes Aluminiumoxid (AlO(OH) (spez. Oberfläche = 300 m$^2$/g; Porenvolumen = 0,46 ml/g) wurde mit 5 Gew.-% SiO$_2$ vermischt und nach Zusatz von 3 Gew.-% Al-Stearat zu Tabletten im Format 4,5×4,5 mm geformt. Die Tabletten wurden mit wässriger PdCl$_2$-Lösung imprägniert, so dass nach der Reduktion (3 Stunden bei 700 K) ein Pd-Gehalt von 0,3 Gew.-% erhalten wurde. Der Katalysator wird 3 Stunden bis zu 775 K unter Luftzutritt calciniert.

Vergleichsbeispiel 2

Die Arbeitsweise von Vergleichsbeispiel 1 wurde mit der Abweichung wiederholt, dass statt SiO$_2$ 5 Gew.-% eines HY-Molekularsiebes zugesetzt wurden.

Vergleichsbeispiel 3

Ein SiO$_2$-Cogel, bei dem 0,3 Gew.-% des Si durch Ti$^{4+}$ (nicht in Form von Titandioxid) ersetzt sind (spez. Oberfläche 300 m$^2$/g) wird mit einer wässrigen PdCl$_2$-Lösung imprägniert, wobei der Pd-Gehalt nach der Reduktion (3 Stunden bei 700 K) 0,25% beträgt. Der Katalysator wird zu Tabletten mit den Abmessungen 4,5×4,5 mm geformt.

Tabelle I
Physikalische und chemische Daten der verwendeten Katalysatoren

| Katalysator | Abmessungen (mm) | BET-Oberfläche (m$^2$/g) | Schüttgewicht (g/l) | Porenvolumen Pd (ml/g) | (Gew.-%) |
|---|---|---|---|---|---|
| Beispiel 1 | 4,5×4,5 | 120±10 | 870±50 | 0,3±0.05 | 0,20±0,05 |
| 2 | 4,5×4,5 | 110±10 | 900±50 | 0,2±0,05 | 0,20±0,05 |
| 3 | 4,5×4,5 | 150±10 | – | 0,2±0,05 | 0,25±0,05 |
| Beispiel 4 | 4,5×4,5 | – | – | – | 0,10±0,05 |
| 5 | 4,5×4,5 | – | – | – | 0,30±0,05 |
| Beispiel 6 | 4,5×4,5 | 120±10 | 870±50 | 0,2±0,05 | 0,20±0,05 |
| 7 | 4,5×4,5 | 120±10 | 870±50 | 0,2±0,05 | 0,20±0,05 |
| 8 | 4,5×4,5 | 100±10 | 900±50 | 0,2±0,05 | 0,20±0,05 |
| Beispiel 9 | Pulver | 50 | – | 0,0 | 0,56 |
| 10 | Pulver | 50 | – | 0,0 | 1,19 |
| Vergleichsbeisp. 1 | 4,5×4,5 | – | – | – | 0,30±0,05 |
| 2 | 4,5×4,5 | – | – | – | 0,30±0,05 |
| 3 | 4,5×4,5 | 300±20 | – | – | 0,25±0,05 |
| (A) γ Al$_2$O$_3$+0,5% Pd 0,5% Cr | 4,5×4,5 T | 180±10 | 900±50 | 0,17±0,05 | 0,5±0,05 |
| (B) γ Al$_2$O$_3$+0,3% Pd | 3–5 S | 95±5 | 650±50 | 0,57±0,05 | 0,3±0,05 |
| (C) Al$_2$O$_3$+0,3% Pd | 4,5×4,5 J | 190±10 | 850±50 | 0,14±0,05 | 0,3±0,05 |
| (D) Al$_2$O$_3$ + 0,5% Pd | 4,5×4,5 J | 185±10 | 650±50 | 0,14±0,05 | 0,5±0,05 |

(S = Kugeln, T = Tabletten, A–D handelsübliche Katalysatoren)

Die Katalysatoren wurden auf ihre Hydrieraktivität und -selektivität untersucht. Die Ergebnisse sind in den Tabellen II und III angegeben.

Bei der Verwendung pulverförmiger Katalysatoren wurde die Hydrierreaktion in flüssiger Phase in einem 300 ml-Autoklaven durchgeführt. Es wurden 500 mg Katalysator und 100 ml einer Einsatzmischung aus 1-Hexen und 1,5 Hexadien (Gewichtsverhältnis 10:1) verwendet. Während der Reaktion wurde bei 1000 U/min gerührt. Der Druck betrug 5 bar, die Temperatur 348 K.

Tabelle II
Hydrierung eines Gemisches aus 1-Hexen und 1,5-Hexadien

| Katalysator | $t_1$ (min) | $t_2/t_1$ | $S_{1/2}$ |
|---|---|---|---|
| Beispiel 1 | 20 | 25 | 100 |
| Beispiel 2 | 22 | 27 | 100 |
| Beispiel 3 | 16 | 11 | 100 |
| Beispiel 4 | 11 | 14 | 100 |
| Beispiel 5 | 5 | 14 | 100 |
| Beispiel 6 | 30 | 12 | 100 |
| Beispiel 7 | 35 | 14 | 100 |
| Beispiel 8 | 18 | 11 | 100 |
| Beispiel 9 | 30 | 12 | 100 |
| Beispiel 10 | 30 | 11,7 | 100 |
| Vergleichsbeisp. 1 | 15 | 11 | 93 |
| 2 | 10 | 9 | 92 |
| 3 | 93 | 2 | 40 |
| Kat. A | 35 | 4,6 | 76 |
| Kat. B | 95 | 1,9 | 88 |

Anmerkungen:
Die Selektivität S ist definiert durch den prozentualen Anteil von Hexan, gebildet aus 1,5-Hexadien, an der gebildeten Gesamthexanmenge zur Zeit $t_1=1/2$.

$t_1$ ist die Zeit in min bis zur vollständigen 1,5-Hexadienhydrierung.

$t_2$ ist die Zeit in min bis zur vollständigen Hexenhydrierung.

Je grösser das $t_2/t_1$-Verhältnis ist, desto vorteilhafter ist die Hydrieraktivität des Katalysators bei der 1,5-Hexadienhydrierung.

Mit tablettenförmigen Katalysatoren (Abmessungen 4,5×4,5 mm) wurde die Hydrierung von 1-Decen/1,9-Decadienmischungen durchgeführt. Die Kohlenwasserstoffmischungen wurden aus den käuflichen Komponenten hergestellt. Der Diengehalt betrug jeweils etwa 6 Gew.-%. Es wurde ein 1-Liter-Autoklav verwendet. Die eingesetzte Menge betrug 300 ml. Es waren 10 g Katalysator in einem Korb angeordnet, der sich während der Reaktion zwischen flüssiger Phase und Gasphase auf- und abbewegte. Dadurch wurde eine sogenannte «trickle-phase»-Hydrierung simuliert. Der Druck beträg bei den einzelnen Versuchen 5, 10 und 30 bar, die Temperatur 348 und 383 K.

Tabelle III
Hydrierung einer $C_{10}$-Olefin-Diolefin-Mischung

| Katalysator | $t_{1/5}$ (min) | $S_{1/5}$ | $s^+$ | P (bar)/ T (K) |
|---|---|---|---|---|
| Beispiel 1 | 39 | 38 | 30 | 20/348 |
| | 51 | 100 | 78 | 5/373 |
| Beispiel 2 | 37 | 100 | 53 | 20/348 |
| | 43 | 100 | 75 | 10/348 |
| Beispiel 8 | 54 | 100 | 41 | 20/348 |
| Kat. A | 88 | 10 | 38 | 20/348 |
| Kat. B | 22 | 12 | 13 | 20/348 |
| Kat. C | 37 | 7 | 0 | 30/348 |
| Kat. D | 53 | 14 | 47 | 20/348 |

Anmerkungen:
$t_{1/5}$ ist die Zeit, die benötigt wurde, um den ursprünglichen Diolefinanteil (10 Gew.-%) auf 1/5 (2/5) zu reduzieren.

$S_{1/5}$ ist das Verhältnis der prozentualen Diolefinkonversion zur Olefinkonversion zur Zeit $t_{1/5}$. $S > 100$ heisst, dass auch ein Teil des vorhandenen Olefins zu Alkan hydriert wird, nicht nur das Diolefin.

$S^+$ gibt an, welcher prozentuale Anteil des verbrauchten Diolefins zum Olefin umgewandelt wird (nach 10 min Reaktionszeit).

**Patentansprüche**

1. Verfahren zur selektiven katalytischen Hydrierung von mehrfach ungesättigten organischen Verbindungen, gekennzeichnet durch die Verwendung eines Katalysators, enthaltend

(A) eine Metallkomponente aus einem oder mehreren Elementen der Gruppe VIII des Periodensystems auf

(B) einem Trägermaterial auf der Basis

($b_1$) eines oder mehrerer n-halbleitender Oxide eines oder mehrerer Elemente aus den Nebengruppen IVb, Vb und VIb des Periodensystems oder des Thoriums oder Cers, bzw. auf der Basis

($b_2$) eines oder mehrerer n-halbleitender Mischoxide der Formel $Me_2Me_1 (O)_x$, worin $Me_1$ ein Element aus der in ($b_1$) definierten Gruppe, $Me_2$ ein Erdalkalimetall oder ein von $Me_1$ verschiedenes Element aus der in ($b_1$) definierten Gruppe und x die Anzahl der zur Absättigung von $Me_1$ und $Me_2$ bis zum Bereich eines n-halbleitenden Zustandes erforderlichen Sauerstoffatome bedeuten;

wobei das Wasserstoff-Chemisorptionsvermögen, ausgedrückt als das Aomverhältnis zwischen chemisorbierten Wasserstoffatomen und an der Oberfläche der Metallteilchen befindlichen Metallatomen der Metallkomponente (A) ($H/Me_A$) mindestens 0,6:1 beträgt.

2. Verfahren nach Anspruch 1, dadurch gekennzeichnet, dass der Katalysator ein Wasserstoff-Chemisorptionsvermögen ($H/Me_A$) von 0,75 bis 1:1 hat.

3. Verfahren nach Anspruch 1 oder 2, dadurch gekennzeichnet, dass die Metallkomponente (A) des Katalysators ein Edelmetall aus der Gruppe VIII des Periodensystems, insbesondere Palladium, und sein n-halbleitendes Trägermaterial (B) entweder

(b₁) ein Oxid eines der Elemente aus den Nebengruppen IVb, Vb und VIb des Periodensystems, insbesondere von Ti, Zr, V, Nb, Ta, Cr, Mo oder W, oder des Thoriums:

(b₂) SrTiO₃, BaTiO₄ oder TiNb₂O₇ darstellt.

4. Verfahren nach einem der Ansprüche 1 bis 3, dadurch gekennzeichnet, dass der Katalysator neben dem n-halbleitenden Trägermaterial ein inertes, nicht n-halbleitendes Füllmaterial enthält.

5. Verfahren nach Anspruch 4, dadurch gekennzeichnet, dass das inerte, nicht n-halbleitende Füllmaterial des Katalysators Aluminiumoxid darstellt.

6. Verfahren nach einem der Ansprüche 1 bis 5, dadurch gekennzeichnet, dass der Katalysator dadurch erhältlich ist, dass man eine reduzierbare Verbindung der Metallkomponente (A) auf eine Vorstufe des n-halbleitenden Trägermaterials (B) aufbringt und unter Bedingungen, bei denen das Wasserstoff-Chemisorptionsvermögen (H/Me$_A$) des erhaltenen Katalysators auf mindestens 0,6:1 gehalten wird, zur Metallkomponente (A) reduziert.

7. Verfahren nach einem der Ansprüche 1 bis 6, dadurch gekennzeichnet, dass der Katalysator dadurch erhältlich ist, dass man die Reduktion der reduzierbaren Verbindung der Metallkomponente (A) in einer reduzierenden Atmosphäre bei Temperaturen durchführt, bei denen das Wasserstoff-Chemisorptionsvermögen (H/Me$_A$) nicht unter 0,6:1 absinkt.

8. Verfahren nach einem der Ansprüche 1 bis 6, dadurch gekennzeichnet, dass der Katalysator dadurch erhältlich ist, dass man die Reduktion der reduzierbaren Verbindung der Metallkomponente (A) mit einem Reduktionsmittel in flüssiger Phase, vorzugsweise in wässrigem Medium durchführt.

9. Verfahren nach einem der Ansprüche 1 bis 6, dadurch gekennzeichnet, dass der Katalysator dadurch erhältlich ist, dass man die Reduktion der reduzierbaren Verbindung der Metallkomponente (A) in an sich bekannter Weise photochemisch bei Umgebungstemperatur durchführt.

10. Verfahren nach einem der Ansprüche 1 bis 5, dadurch gekennzeichnet, dass der Katalysator dadurch erhältlich ist, dass man eine reduzierbare Verbindung der Metallkomponente (A) auf eine Vorstufe des n-halbleitenden Trägermaterials (B) aufbringt und unter Bedingungen, bei denen das Wasserstoff-Chemisorptionsvermögen des erhaltenen Katalysators (H/Me$_A$) unterhalb 0,6:1 absinkt, zur Metallkomponente (A) reduziert und den erhaltenen Katalysator oxidiert, bis das Wasserstoff-Chemisorptionsvermögen (H/Me$_A$) den Wert von 0,6:1 wieder erreicht oder überschritten hat.

11. Verfahren nach einem der Ansprüche 1–10, gekennzeichnet durch die Verwendung eines mit phosphat- oder fluoridhaltigen Lösungen imprägnierten Katalysators.

12. Verfahren nach einem der Ansprüche 1 bis 11, dadurch gekennzeichnet, dass man als mehrfach ungesättigte organische Verbindungen Kohlenwasserstoffe verwendet.

13. Verfahren nach einem der Ansprüche 1 bis 11, dadurch gekennzeichnet, dass man als mehrfach ungesättigte organische Verbindungen Diolefine mit isolierten Doppelbindungen bzw. die in Kohlenwasserstoffgemischen enthaltenen Diolefine mit isolierten Doppelbindungen verwendet.

14. Verfahren nach einem der Ansprüche 1 bis 11, dadurch gekennzeichnet, dass man als mehrfach ungesättigte Kohlenwasserstoffe Diene mit kumulierten und/oder konjugierten Doppelbindungen verwendet.

15. Verfahren nach einem der Ansprüche 1 bis 14, dadurch gekennzeichnet, dass man mehrfach ungesättigte organische Verbindungen verwendet, die neben C=C-Doppelbindungen auch C=O- oder C=N-Gruppen enthalten.

**Revendications**

1. Procédé pour l'hydrogénation catalytique sélective de composés polyinsaturés, caractérisé par l'utilisation d'un catalyseur contenant:

(A) un constituant métallique formé d'un ou plusieurs éléments du groupe VIII de la Classification Périodique sur

(B) un support à base

(b₁) d'un ou plusieurs oxydes semi-conducteurs du type n d'un ou plusieurs éléments des sous-groupes IVb, Vb et VIb de la Classification Périodique ou du thorium ou du cérium, ou à base

(b₂) d'un ou plusieurs oxydes mixtes semi-conducteurs du type n de la formule Me$_2$Me$_1$ (O)$_x$ dans laquelle Me$_1$ désigne un élément du groupe défini en (b₁), Me$_2$ un métal alcalino-terreux ou un élément, différent de Me$_1$, du groupe défini en (b₁) et x le nombre des atomes d'oxygène nécessaires pour saturer Me$_1$ et Me$_2$ jusqu'au domaine d'un état semi-conducteur du type n; le pouvoir de chimisorption d'hydrogène, exprimé comme le rapport atomique (H/Me$_A$) entre atomes d'hydrogène chimisorbé et atomes de métal du constituant métallique (A) situés à la surface des particules métalliques, étant d'au moins 0,6:1.

2. Procédé selon la revendication 1, caractérisé par le fait que le catalyseur a un pouvoir de chimisorption d'hydrogène (H/Me$_A$) de 0,75 à 1:1.

3. Procédé selon l'une des revendications 1 et 2, caractérisé par le fait que son constituant métallique (A) est un métal noble du groupe VIII de la Classification Périodique, en particulier le palladium, et que son support semi-conducteur du type n (B) est:

(b₁) soit un oxyde de l'un des éléments des sous-groupes IVb, Vb et VIb de la Classification Périodique, en particulier de Ti, Zr, V, Nb, Ta, Cr, Mo ou W, ou du thorium:

(b₂) soit SrTiO₃, BaTiO₄ ou TiNb₂O₇.

4. Procédé selon l'une des revendications 1 à 3, caractérisé en ce que le catalyseur contient, outre le support semiconducteur du type n, une charge inerte non semiconductrice du type n.

5. Procédé selon la revendication 4, caractérisé en ce que la charge inerte non semiconductrice du type n̲ du catalyseur constitue l'oxyde d'aluminium.

6. Procédé selon l'une des revendications 1 à 5, caractérisé en ce que le catalyseur peut être obtenu en appliquant un composé réductible du constituant métallique (A), par exemple une solution d'un sel de métal noble, sur un progéniteur du support semiconducteur du type n̲ (B) en réduisant un constituant métallique (Ā) dans des conditions dans lesquelles le pouvoir de chimisorption d'hydrogène ($H/Me_A$) du catalyseur obtenu est maintenu à au moins 0,6:1.

7. Procédé selon l'une des revendications 1 à 6, caractérisé en ce que l'on peut obtenir le catalyseur en effectuant la réduction du composé réductible du constituant métallique (A) dans une atmosphère réductrice à des températures auxquelles le pouvoir de chimisorption d'hydrogène ($H/Me_A$) ne s'abaisse pas en dessous de 0,6:1.

8. Procédé selon l'une des revendications 1 à 6, caractérisé en ce que l'on peut obtenir le catalyseur en effectuant la réduction du composé réductible du constituant métallique (A) avec un réducteur, en phase liquide, de préférence en milieu aqueux.

9. Procédé selon l'une des revendications 1 à 6, caractérisé en ce que l'on peut obtenir le catalyseur en effectuant photochimiquement la réduction du composé réductible du constituant métallique (A), de manière en elle-même connue, à la température ambiante.

10. Procédé selon l'une des revendications 1 à 5, caractérisé en ce que l'on peut préparer le catalyseur utilisé selon l'invention en appliquant un composé réductible du constituant métallique (A) sur un progéniteur du support semi-conducteur du type n̲ (B) et en réduisant un constituant métallique (Ā) dans des conditions dans lesquelles le pouvoir de chimisorption d'hydrogène du catalyseur obtenu ($H/Me_A$) s'abaisse en dessous de 0,6:1 et en oxydant le catalyseur obtenu jusqu'à ce que le pouvoir de chimisorption d'hydrogène ($H/Me_A$) ait à nouveau atteint ou dépassé la valeur de 0,6:1.

11. Procédé selon l'une des revendications 1 à 10, caractérisé par l'utilisation d'un catalyseur imprégné de solutions contenant du phosphate ou du fluorure.

12. Procédé selon l'une des revendications 1 à 11, caractérisé en ce que, comme composés organiques polyinsaturés, on utilise des hydrocarbures.

13. Procédé selon l'une des revendications 1 à 11, caractérisé en ce que, comme composés organiques polyinsaturés, on utilise des dioléfines à doubles liaisons isolées ou les dioléfines à doubles liaisons isolées contenues dans des mélanges d'hydrocarbures.

14. Procédé selon l'une des revendications 1 à 11, caractérisé en ce que, comme hydrocarbure polyinsaturés, on utilise des diènes à doubles liaisons jumelées et/ou conjuguées.

15. Procédé selon l'une des revendications 1 à 14, caractérisé en ce que l'on utilise des composés organiques polyinsaturés qui, outre des doubles liaisons C=C, contiennent aussi des groupes C=O ou C=N.

**Claims**

1. A method for the selective catalytic hydrogenation of polyunsaturated organic compounds, characterised by the use of a catalyst, containing

(A) a metal component of one or more elements of group VIII of the periodic system on

(B) a support material based on

($b_1$) one or more n-semiconductive oxides of one or more elements from sub-groups IVb, Vb and VIb of the periodic system or of thorium, or cerium, and/or based on

($b_2$) one or more n-semiconductive mixed oxides having the formula $Me_2Me_1(O)_x$, wherein $Me_1$ signifies an element of the group defined in ($b_1$), $Me_2$ an alkaline earth metal or an element different to $Me_1$ from the group defined in ($b_1$) and x the number of oxygen atoms required to saturate $Me_1$ and $Me_2$ up to the region of an n-semiconductive state;

wherein the hydrogen chemisorption capacity, expressed as the atomic ratio between chemisorbed hydrogen atoms and metal atoms of the metal component (A) situated on the surface of the metallic particles, ($H/Me_A$) is at least 0,6:1.

2. A method according to Claim 1, characterised in that the catalyst has a hydrogen chemisorption capacity ($H/Me_A$) of from 0,75 to 1:1.

3. A method according to Claim 1 or 2, characterised in that the metal component (A) of the catalyst represents a noble metal from group VIII of the periodic system, in particular palladium, and n-semiconductive support material (B) represents either

($b_1$) an oxide of one of the elements from sub-groups IVb, Vb and VIb of the periodic system, in particular of Ti, Zr, V, Nb, Ta, Cr, Mo or W, or of thorium;

($b_2$) $SrTiO_3$, $BaTiO_4$ or $TiNb_2O_7$.

4. A method according to any one of Claims 1 to 3, characterised in that in addition to the n-semiconductive support material the catalyst contains an inert non-n-semiconductive filler.

5. A method according to Claim 4, characterised in that the inert non-n-semiconductive filler of the catalyst is aluminium oxide.

6. A method according to any one of Claims 1 to 5, characterised in that the catalyst can be obtained by applying a reducible compound of metal component (A) on to a preliminary stage of the n-semiconductive support material (B) and, under conditions in which the hydrogen chemisorption capacity ($H/Me_A$) of the resultant catalyst is maintained at at least 0,6:1, reducing it to the metal component (A).

7. A method according to any one of Claims 1 to 6, characterised in that the catalyst can be obtained by carrying out the reduction of the reducible compound of metal component (A) in a reducing atmosphere at temperatures at which the hydrogen chemisorption capacity (H/Me$_A$) dose not drop below 0,6:1.

8. A method according to any one of Claims 1 to 6, characterised in that the catalyst can be obtained by carrying out the reduction of the reducible compound of metal component (A) with a reducing agent in liquid phase, preferably in an aqueous medium.

9. A method according to any one of Claims 1 to 6, characterised in that the catalyst can be obtained by carrying out the reduction of the reducible cpound of metal component (A) photochemically at ambient temperature in a manner known per se.

10. A method according to any one of Claims 1 to 5, characterised in that catalyst can be obtained by applying a reducible compound of metal component (A) on to a preliminary stage of the n-semiconductive support material (B) and, under conditions in which the hydrogen chemisorption capacity of the resultant catalyst (H/Me$_A$) drops below 0,6:1, reducing it to the metal component (A) and oxidising the resultant catalyst until the hydrogen chemisorption capacity (H/Me$_A$) has again reached or exceeded the value of 0,6:1.

11. A method according to any one of Claims 1 to 10, characterised by the use of a catalyst impregnated with solutions containing phosphate or fluoride.

12. A method according to any one of Claims 1 to 11, characterised in that hydrocarbons are used as polyunsaturated organic compounds.

13. A method according to any one of Claims 1 to 11, characterised in that diolefins with isolated double bonds and/or the diolefins with isolated double bonds contained in hydrocarbon mixtures are used as polyunsaturated organic compounds.

14. A method according to any one of Claims 1 to 11, characterised in that dienes with cumulative and/or conjugate double bonds are used as polyunsaturated hydrocarbons.

15. A method according to any one of Claims 1 to 14, characterised in that polyunsaturated organic compounds are used which in addition to C=C-double bonds also comprise C=O- or C=N-groups.